# EUROPEAN PATENT SPECIFICATION

(11) **EP 2 896 364 B1**
(45) Date of publication and mention of the grant of the patent: **15.01.2020**
(21) Application number: 12869581.4
(22) Date of filing: 30.10.2012
(51) Int. Cl.: A61B 6/00, G01T 1/20, H04N 5/32

(54) **X-RAY IMAGE PHOTOGRAPHING APPARATUS AND METHOD THEREOF**
RÖNTGENBILDAUFNAHMEVORRICHTUNG UND VERFAHREN DAFÜR
APPAREIL DE PHOTOGRAPHIE D'IMAGE RADIOLOGIQUE ET SON PROCÉDÉ

(30) Priority: 11.09.2012 KR 20120100639
(43) Date of publication of application: 22.07.2015
(73) Proprietor: Vieworks Co. Ltd., Gyeonggi-do 462-736 (KR)
(72) Inventor: SUNG, Yong Hak, Seongnam-si Gyeonggi-do 462-120 (KR); KIM, Tae Kyun, Seongnam-si Gyeonggi-do 462-120 (KR)
(74) Representative: Zacco Sweden AB
(86) International application number: PCT/KR2012/008992
(87) International publication number: WO 2014/042310

(56) References cited:
- EP-A1- 2 449 967
- GB-A- 2 304 017
- JP-A- 2005 177 379
- JP-A- 2007 258 149
- KR-A- 20110 080 363
- KR-B1- 101 126 582
- KR-B1- 101 126 582
- KR-B1- 880 000 823
- US-A1- 2012 199 751

## Description

### [Technical Field]

The present invention relates to an X-ray imaging apparatus and method, and more particularly, to an X-ray imaging apparatus and method, which can improve reliability of an X-ray imaging operation by verifying validity of an automatic exposure request signal while an X-ray image is captured in accordance with the automatic exposure request signal generated by detecting an X-ray emitted from an X-ray generator.

### [Background Art]

In general, an X-ray diagnosis apparatus includes an X-ray generator that generates and emits an X-ray to an object, and an X-ray imaging apparatus that captures an image of the X-ray passed through the object.

The X-ray imaging apparatus of such an X-ray diagnosis apparatus sequentially performs a flushing operation for flushing dark current accumulated in an interior image sensor every line unit, an exposure operation for absorbing an X-ray emitted from the X-ray generator, and a readout operation for allowing the X-ray imaging apparatus to read electric charges generated by emission of the X-ray and output image data.

Here, the X-ray imaging apparatus receives an exposure request signal from the X-ray generator during the flushing operation, and transmits an exposure ready signal to indicate that exposure preparation has been completed to the X-ray generator after completing the flushing operation throughout an overall area, thereby performing the exposure operation.

Thus, a trigger type for handshaking condition signals between the X-ray generator and the X-ray imaging apparatus is called an active line trigger type.

In the case of using such an active line trigger type X-ray generator, the X-ray imaging apparatus can obtain image data of good quality since it can complete the flushing operation for a period of time between a time point of receiving the exposure request signal and a time point of transmitting the exposure ready signal.

However, in the case of using a passive line trigger type X-ray generator that emits X-rays after transmitting only the exposure request signal to the X-ray imaging apparatus or a non-line trigger type X-ray generator that emits X-rays without transmitting the exposure request signal to the X-ray imaging apparatus, time taken in converting the X-ray imaging apparatus from the flushing operation to the exposure operation causes some loss of the emitted X-rays, thereby making it difficult to obtain image data of good quality.

Also, if the exposure operation is performed to reduce the loss of X-rays in the state that the flushing operation for a line in some areas is not completed in the X-ray imaging apparatus, output image data can suffer from quality deterioration in a unit of line.

Accordingly, to solve such a problem, there has been proposed a method of minimizing X-ray loss to obtain image data of good quality in an interface environment, in which X-rays are asynchronously emitted, by detecting the X-rays emitted from the X-ray generator and generating an automatic exposure request signal for automatically indicating the X-ray emission.

KR 101 126 582 B1 discloses a prior art X-ray imaging apparatus capable of generating a trigger signal through detection of radiated X-rays.

In this type, a circuit for generating an automatic exposure request signal includes an amplification circuit so as to generate the automatic exposure request signal even though a light detector or an X-ray detector detects a low level signal.

However, there is a problem that noise signals such as impulses can be amplified, thereby unintentionally generating the automatic exposure request signal.

In addition, in operation of the X-ray imaging apparatus, rapid temperature change can cause offset change due to variation in conditions of elements constituting the circuit for generating the trigger signal, thereby unintentionally generating the automatic exposure request signal.

Further, the X-ray imaging apparatus is frequently brought into contact with a patient for diagnosis or the like. Vibration due to such contact can change the conditions of the light detector or the X-ray detector, thereby unintentionally generating the automatic exposure request signal.

EP, 2 449 967 A1 discloses a radiation detecting device comprising an acceleration sensor to detect a magnitude of a shake or shock during an X-ray exposure in order to check the validity of an X-ray exposure start signal.

US 2012/199751 A1 discloses a radiation image detecting device for detecting start of irradiation. Validity of an X-ray exposure start signal is determined based on a comparison of two voltage signals generated from pixels of a flat panel detector.

GB 2 304 017 A discloses an imaging apparatus wherein threshold conditions for determining false triggering are adapted to ambient conditions.

Meanwhile, in order to obtain good quality when recapturing an X-ray image, it is necessary for the X-ray imaging apparatus to secure a preparation time for recapturing the X-ray image after capturing an X-ray image. Thus, the active line trigger type X-ray imaging apparatus secures a predetermined period of preparation time for recapturing.

However, a non-line trigger type X-ray imaging apparatus has a problem in that X-rays can be emitted regardless of the conditions of the X-ray imaging apparatus.

That is, if X-rays are emitted for X-ray imaging within a very short period of time in a non-stabilized state of the image detector of the X-ray imaging apparatus, the X-ray imaging apparatus can fail to generate a valid automatic exposure request signal or to obtain an X-ray image of good quality.

### [Summary]

The present invention is conceived to solve such problems in the art, and an aspect of the present invention is to provide an X-ray imaging apparatus, which can prevent an X-ray image from being captured by an unintentional automatic exposure request signal, thereby improving reliability of X-ray imaging operation and X-ray images obtained thereby.

Another aspect of the present invention is to provide an X-ray imaging apparatus, which can rapidly stabilize an image detector by adjusting bias voltage applied to the image detector and controlling a gate driver and a readout unit at high speed, thereby shortening a preparation time for recapturing and thus preventing loss of X-rays.

In accordance with one aspect of the present invention, an X-ray imaging apparatus includes: a scintillator panel which converts an X-ray emitted from an X-ray generator into visible light; an image detector which includes a plurality of pixels arranged in a matrix, and charges the plurality of pixels with electric charges proportional to intensity of the visible light converted by the scintillator panel; a gate driver which selects a line of the image detector and applies a drive signal to pixels of the selected line; an automatic exposure request signal generator which detects the X-ray emitted from the X-ray generator and generates an automatic exposure request signal; and a controller which verifies validity of the automatic exposure request signal upon receiving the automatic exposure request signal from the automatic exposure request signal generator, and controls a time point for exposure operation according to states of the drive signal when it is determined that the automatic exposure request signal is valid. The X-ray imaging apparatus further comprises the features represented in the characterizing portion of claim 1.

The apparatus may further include: a vibration sensor for detecting external vibration, wherein the controller determines that the automatic exposure request signal is valid when vibration input from the vibration sensor is less than or equal to reference vibration.

The controller may determine that the automatic exposure request signal is valid when the automatic exposure request signal is continuously input for a reference period of time.

According to the present invention, when capturing an X-ray image using an automatic exposure request signal generated through detection of X-rays emitted from an X-ray generator, the X-ray imaging apparatus may verify validity of the automatic exposure request signal, thereby preventing unintentional generation of the automatic exposure request signal due to vibration, temperature change, noise, etc.

In addition, according to the present invention, the X-ray imaging apparatus may rapidly stabilize an image detector by adjusting bias voltage applied to the image detector and controlling a gate driver and a readout unit at high speed, thereby shortening a preparation time for recapturing and thus generating a valid automatic exposure request signal even though recapturing is carried out within a very short period of time.

Thus, according to the present invention, the X-ray imaging apparatus may prevent unintentional generation of an automatic exposure request signal and reduce a preparation time for recapturing, thereby improving reliability of an X-ray imaging operation and X-ray images obtained thereby.

### [Brief Description of Drawings]

Fig. 1 is a block diagram of an X-ray imaging apparatus according to one exemplary embodiment.
Fig. 2 is a view of a configuration for performing a gate selection operation and a readout operation in the X-ray imaging apparatus according to the exemplary embodiment.
Fig. 3 is a view hierarchically showing the X-ray imaging apparatus according to the exemplary embodiment.
Fig. 4 is a top view of a position where an automatic exposure request signal generator is placed in the X-ray imaging apparatus according to the exemplary embodiment.
Fig. 5 is a first example showing the configuration of the automatic exposure request signal generator in the X-ray imaging apparatus according to the exemplary embodiment of the present invention.
Fig. 6 is a second example showing the configuration of the automatic exposure request signal generator in the X-ray imaging apparatus according to the exemplary embodiment.
Fig. 7 is a third example showing the configuration of the automatic exposure request signal generator in the X-ray imaging apparatus according to the exemplary embodiment.
Fig. 8 is an example showing a structure for holding the automatic exposure request signal generator in the X-ray imaging apparatus according to the exemplary embodiment of the present invention.
Fig. 9 is a flowchart of an X-ray imaging method according to one exemplary embodiment.
Fig. 10 is a flowchart of an X-ray imaging method according to another exemplary embodiment.
Fig. 11 is a flowchart of an X-ray imaging method according to a further exemplary embodiment.
Fig. 12 is a flowchart of a high-speed a flushing operation by adjusting bias voltage applied to an image detector and controlling a gate driver and a readout unit at high speed, in the X-ray imaging method according to the exemplary embodiment.

### [Detailed Description]

Hereinafter, exemplary embodiments will be described with reference to the accompanying drawings. It should be noted that the drawings are not to precise scale and may be exaggerated in thickness of lines or size of components for descriptive convenience and clarity only. Furthermore, the terms used herein are defined by taking functions of the present disclosure into account and can be changed according to user or operator's custom or intention. Therefore, definition of the terms should be made according to the overall disclosure set forth herein.

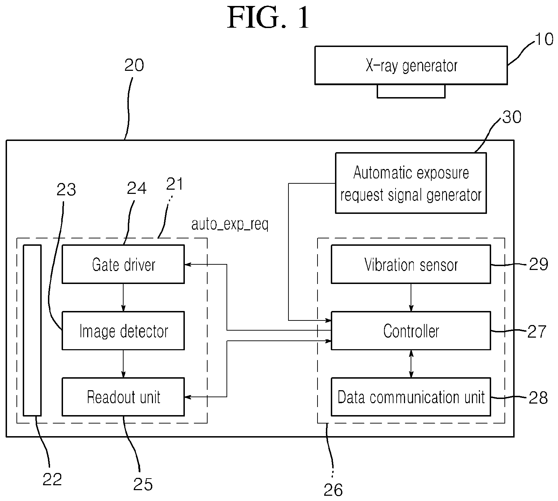

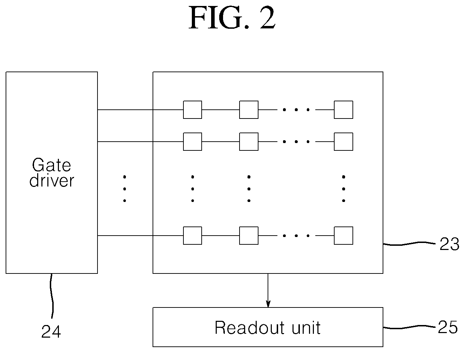

Fig. 1 is a block diagram of an X-ray imaging apparatus according to one exemplary embodiment and Fig. 2 is a view of a configuration for performing a gate selection operation and a readout operation in the X-ray imaging apparatus according to the exemplary embodiment.

An X-ray imaging apparatus 20 detects an X-ray having passed through an object 40 and obtains X-ray image data of the object 40.

Referring to Figs. 1 and 2, the X-ray imaging apparatus 20 according to one exemplary embodiment includes an image sensing unit 21, an electronic circuit board 26, and an automatic exposure request signal generator 30.

The image sensing unit 21 includes a scintillator panel 22, an image detector 23, a gate driver 24, and a readout unit 25.

The scintillator panel 22 converts an X-ray emitted from the X-ray generator 10 into visible light.

The image detector 23 detects the visible light converted by the scintillator panel 22. As shown in Fig. 2, the image detector 23 includes a plurality of pixels arranged in a matrix and charges the respective pixels with electric charges proportional to the intensity of the visible light.

The gate driver 24 selects a certain line of the image detector 23 in sequence and applies a drive signal to the selected line according to control of the controller 27.

When the gate driver 24 applies a drive signal to a certain line, the readout unit 25 described below may read out an electric charge level in each pixel of the corresponding line. Here, the drive signal applied by the gate driver 24 may be a voltage signal for turning on a thin film transistor (TFT) connected to each pixel of the line.

When the gate driver 24 applies the drive signal to a certain line of the image detector 23, the readout unit 25 reads out an electric charge level charged in each pixel of the line.

At this point, when performing a flushing operation, the readout unit 25 discards an electric charge level charged in each pixel instead of reading out the electric charge level, thereby removing dark current accumulated in each pixel of the image detector 23.

On the other hand, when performing a readout operation, the readout unit 25 reads out the electric charge level of each pixel and obtains analog data in the form of voltage, followed by converting the analog data into digital data and transmitting the digital data to the controller 27.

Then, the controller 27 combines the digital data received from the readout unit 25 in a unit of line and transmits the combined digital data to a personal computer (PC) through a data communication unit 29.

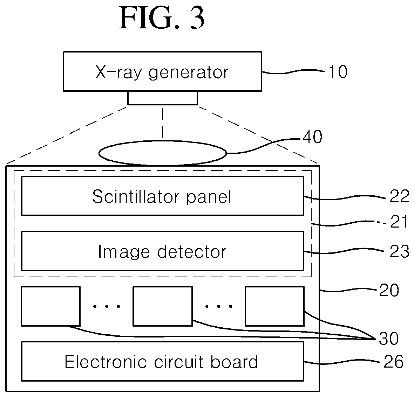

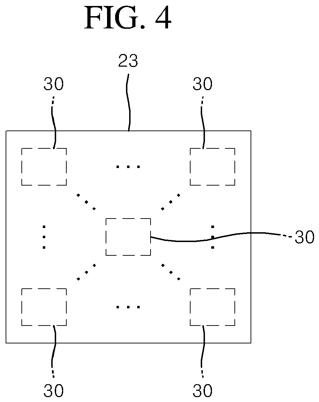

Fig. 3 is a view hierarchically showing the X-ray imaging apparatus according to the exemplary embodiment and Fig. 4 is a top view of a position where an automatic exposure request signal generator is placed in the X-ray imaging apparatus according to the exemplary embodiment.

The automatic exposure request signal generator 30 detects an X-ray emitted from the X-ray generator 10 and generates an automatic exposure request signal auto_exp_req as a trigger signal for indicating X-ray emission, thereby transmitting the automatic exposure request signal auto_exp_req to the controller 27.

The automatic exposure request signal generator 30 may be disposed within the X-ray imaging apparatus 20, and may be placed under the image sensing unit 21 including the scintillator panel 22 and the image detector 23 with respect to a direction of emitting X-rays, as shown in Fig. 3.

Also, the automatic exposure request signal generator 30 may be placed on the electronic circuit board 26, on which the controller 27 and the data communication unit 28 are mounted.

As a result, the automatic exposure request signal generator 30 may detect X-rays having passed through the scintillator panel 22 and the image detector 23, and generate the automatic exposure request signal auto exp req.

In this way, the automatic exposure request signal generator 30 is placed within the X-ray imaging apparatus 20 to achieve an integrated system without any separate interface, thereby improving user convenience and enabling detection of X-rays regardless of an X-ray emission range.

As shown in Fig. 4, a plurality of the automatic exposure request signal generators 30 may be placed at locations corresponding to the middle portion and corners of the image sensing unit 21 which includes the scintillator panel 22 and the image detector 23.

As such, since the plurality of automatic exposure request signal generators 30 are placed at many locations within the X-ray imaging apparatus 20, the automatic exposure request signal auto exp req can be advantageously generated by the automatic exposure request signal generators 30 placed at the corners without being affected by the object 40 even through transmittance of the X-ray is decreased at the middle portion by the object 40.

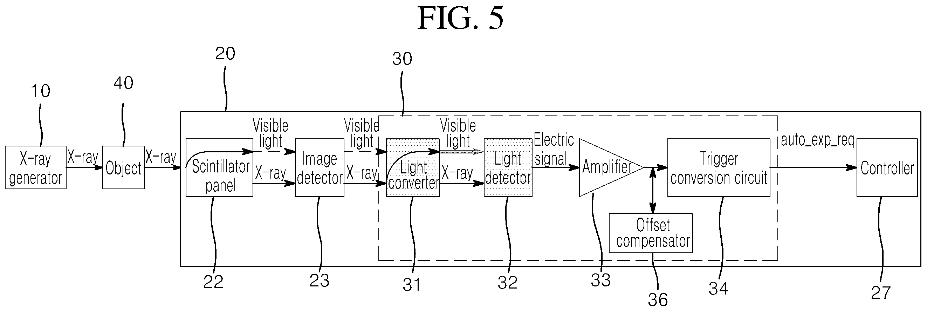

Fig. 5 is a first example showing the configuration of the automatic exposure request signal generator in the X-ray imaging apparatus according to the exemplary embodiment.

As shown in Fig. 5, the automatic exposure request signal generator 30 may include a light converter 31, a light detector 32, an amplifier 33, a trigger conversion circuit 34, and an offset compensator 36.

The light converter 31 is attached to a front side of the light detector 32, and converts an X-ray emitted from the X-ray generator 10 into visible light. The light converter 31 may include various materials, such as a scintillator and the like, which are capable of converting X-rays into visible light.

The light detector 32 detects the visible light converted by the light converter 31 and converts the visible light into an electric signal. The light detector 32 may include various elements such as a photodiode and the like, which are capable of detecting visible light.

Here, the surface of the light detector 32 and a lead through which electric signals are transmitted are shielded to minimize introduction of noise.

The amplifier 33 amplifies the electric signal converted by the light detector 32 and transmits the amplified electric signal to the trigger conversion circuit 34. Then, trigger conversion circuit 34 converts the signal amplified by the amplifier 33 into a digital signal, generates an automatic exposure request signal auto exp req to be used as a trigger signal for indicating X-ray emission, and transmits the automatic exposure request signal to the controller 27.

According to the present invention, the automatic exposure request signal auto_exp_req functioning as the trigger signal is generated by detecting an X-ray emitted from the X-ray generator, so that an X-ray image of good quality can be captured even in the case of using a non-line trigger type X-ray generator that does not support the line trigger type.

Meanwhile, the automatic exposure request signal generator 30 according to one embodiment may set a threshold voltage to determine validity of the electric signal converted by the light detector 32. Accordingly, in an ordinary standby state except for the case of reacting with the emitted X-ray, an offset is adjusted such that the signal having passed through the amplifier 33 can have a voltage less than or equal to the threshold voltage.

However, rapid temperature change can frequently occur in practical use of the X-ray imaging apparatus 20 and the conditions of various electronic elements constituting the automatic exposure request signal generator 30 can also be changed, thereby causing change of the offset.

Accordingly, the offset compensator 36 compensates for the offset of the amplifier 33 so that an output signal of the amplifier 33 can have a voltage less than or equal to the threshold voltage in an ordinary standby state, in which no X-ray is emitted.

As such, the offset compensator 36 prevents unintentional generation of the automatic exposure request signal auto_exp_req when the offset is varied depending on rapid temperature change in the conditions in which the X-ray imaging apparatus 20 is used.

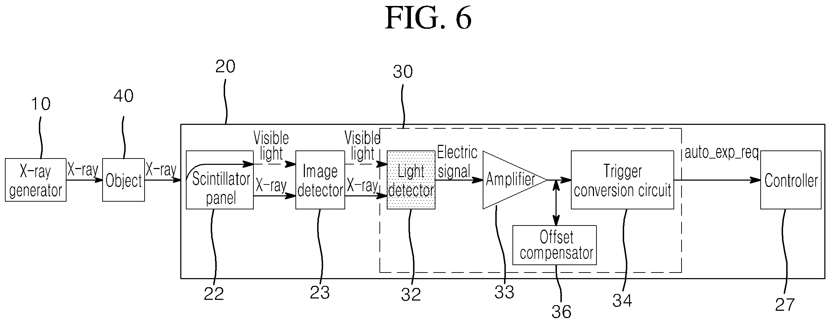

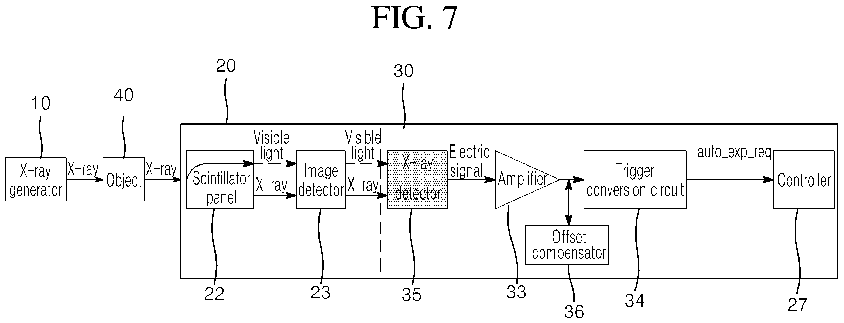

Fig. 6 is a second example showing the configuration of the automatic exposure request signal generator in the X-ray imaging apparatus according to the exemplary embodiment and Fig. 7 is a third example showing the configuration of the automatic exposure request signal generator in the X-ray imaging apparatus according to the exemplary embodiment.

As shown in Fig. 6, the automatic exposure request signal generator 30 may not include the light converter 31.

That is, through the light detector 32, the automatic exposure request signal generator 30 may directly detect visible light emitted from the scintillator panel 22, which absorbs an X-ray emitted from the X-ray generator 10, and may generate an automatic exposure request signal auto exp req.

In addition, as shown in Fig. 7, the automatic exposure request signal generator 30 may include an X-ray detector 35 instead of the light converter 31 and the light detector 32 in order to directly detect an emitted X-ray.

Specifically, the automatic exposure request signal generator 30 uses the X-ray detector 35 to detect an X-ray having passed through the scintillator panel 22 and the image detector 23, and to generate the automatic exposure request signal auto_exp_req. Here, the X-ray detector 35 may include any element capable of detecting an X-ray.

As in the above embodiment, the surface of the light detector 32 and the lead through which the electric signal is transmitted may be shielded to minimize introduction of noise.

Referring back to Fig. 1, the electronic circuit board 26 is a board on which electronic circuit modules are mounted, and includes a controller 27, a data communication unit 28, and a vibration sensor 29.

The vibration sensor 29 detects vibration applied to the X-ray imaging apparatus 20 and sends a vibration signal to the controller 27. Although the vibration sensor 29 is illustrated as being mounted on the electronic circuit board 26 in this embodiment, the present invention is not limited thereto. Alternatively, the vibration sensor 29 may be placed at a portion on which the automatic exposure request signal generator 30 is mounted.

The controller 27 controls the gate driver 24 and the readout unit 25 to control general operation for obtaining image data based on X-rays emitted from the X-ray generator 10. That is, the controller 27 controls general processes for flushing, exposure and a readout operations of the X-ray imaging apparatus 20.

In particular, when the automatic exposure request signal auto_exp_req is received from the automatic exposure request signal generator 30, the controller 27 verifies validity of the automatic exposure request signal auto exp req and controls exposure operation to be performed after a flushing operation is completed with regard to the line to which the gate driver 24 applies the drive signal, if it is determined that the automatic exposure request signal auto exp req is valid.

The controller 27 may verify the validity of the automatic exposure request signal auto exp req using various methods. Here, the tem validity means whether the automatic exposure request signal auto exp req received from the automatic exposure request signal generator 30 is a normal automatic exposure request signal auto exp req generated by X-ray emission.

First, the controller 27 determines that the automatic exposure request signal auto exp req is valid only when vibration sensed by the vibration sensor 29 is less than or equal to predetermined reference vibration.

Here, the reference vibration is a reference value for determining whether variation is large enough to allow the automatic exposure request signal generator 30 to generate the automatic exposure request signal auto exp req even in the case where an X-ray is not emitted, and may be variously set according to designer intention and product specifications as.

With this method, it is possible to prevent the automatic exposure request signal auto_exp_req from being unintentionally generated as the conditions of the light detector or the X-ray detector are varied due to vibration caused by contact between the X-ray imaging apparatus 20 and a patient.

Second, the controller 27 may determine that the automatic exposure request signal auto exp req is valid when the automatic exposure request signal auto exp req is continuously input for a reference period of time.

Here, the reference period of time is a period of time for distinguishing the automatic exposure request signal auto exp req from impulse or noise signals, and may be variously set according to designer intention and product specifications.

With this method, it is possible to prevent the automatic exposure request signal auto exp req from being unintentionally generated as impulse or noise signals are amplified.

Third, if there is a plurality of automatic exposure request signal generators 30, the controller 27 determines that the automatic exposure request signal auto exp req is valid only when a predetermined reference number or more of automatic exposure request signals auto exp req are simultaneously received from the plurality of automatic exposure request signal generators 30.

Here, the reference number refers to the minimum number of automatic exposure request signals auto_exp_req actually generated by X-ray emission, and may be variously set according to the installation locations and numbers of the automatic exposure request signal generators 30.

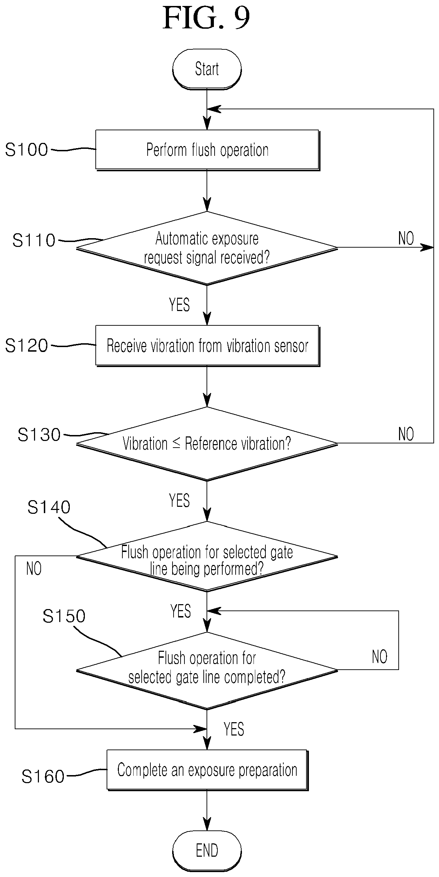

Thus, a specific operation of the controller 27 that verifies the validity of the automatic exposure request signal auto exp req will be described later with reference to Figs. 9 to 11.

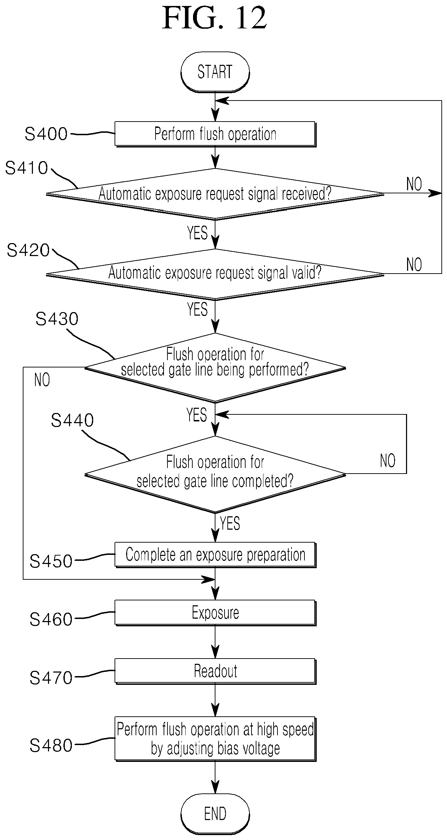

Meanwhile, in the flushing operation, the controller 27 controls the gate driver 24 and the readout unit 25 at high speed, adjusts bias voltage applied to a TFT of each of pixels constituting the image detector 23, thereby allowing the flushing operation to be performed at high speed. This operation will be described below in more detail with reference to Fig. 12.

The data communication unit 29 transmits image data, which is combined in a unit of line through the readout operation by the controller 27, to a personal computer (not shown).

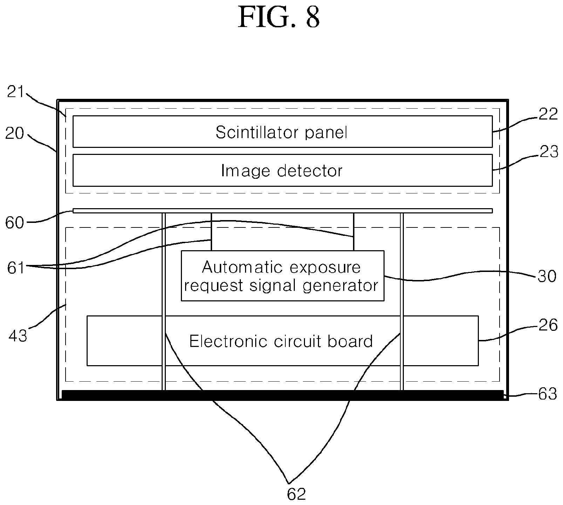

Fig. 8 is an example showing a structure for holding the automatic exposure request signal generator in the X-ray imaging apparatus according to the exemplary embodiment.

The internal structure of the X-ray imaging apparatus 20 according to the exemplary embodiment may be configured, as shown in Fig. 8.

Referring to Fig. 8, a first plate 60 is interposed between the image sensing unit 21 and the electronic circuit board 26 within a housing of the X-ray imaging apparatus 20, and is secured to a second plate 63 placed on a bottom surface of the housing via plate supporter 62.

The automatic exposure request signal generator 30 may be fastened to the first plate 60 through an internal supporter 61 and placed on the electronic circuit board 26.

Thus, when the automatic exposure request signal generator 30 is fixedly supported by the first plate 60, it is possible to minimize transfer of vibration to the automatic exposure request signal generator 30 even though vibration or shock is applied from the exterior to the X-ray imaging apparatus 20.

Fig. 9 is a flowchart of an X-ray imaging method according to one exemplary embodiment of the present invention.

As shown in Fig. 9, the controller 27 controls the gate driver 24 and the readout unit 25 to sequentially select lines of the image detector 23, and performs a flushing operation for flushing dark currents accumulated in a selected line (S100).

Then, the controller 27 checks whether an automatic exposure request signal auto_exp_req is received from the automatic exposure request signal generator 30 (S110).

When the automatic exposure request signal auto exp req is received from the automatic exposure request signal generator 30, the controller 27 receives vibration from the vibration sensor 29 (S120).

Then, the controller 27 determines whether the vibration input from the vibration sensor 29 is less than or equal to predetermined reference vibration (S130).

When the input vibration from the vibration sensor 29 is less than or equal to the predetermined reference vibration, it is determined that the automatic exposure request signal auto_exp_req is not unintentionally generated by the vibration. Thus, the controller 27 determines that the automatic exposure request signal auto exp req is valid.

On the other hand, when the input vibration from the vibration sensor 29 is higher than the predetermined reference vibration, it is determined that the automatic exposure request signal auto exp req is unintentionally generated by the vibration. Thus, the controller 27 returns to the flushing operation (S100) without performing exposure operation.

If it is determined that the automatic exposure request signal auto exp req is valid, the controller 27 determines whether a drive signal is applied to the pixel of the line selected by the gate driver 24 and whether the flushing operation is being performed (S140). That is, the controller 27 determines whether the drive signal applied to the selected line is activated.

If it is determined that the drive signal is applied to the pixel of the selected line and the flushing operation is being performed, the controller 27 determines whether the flushing operation for the selected line is terminated (S150).

When the flushing operation for the selected line is terminated, the controller 27 prepares for exposure operation and performs the exposure operation (S160).

That is, when the drive signal is applied to the pixel of the selected line and the flushing operation is being performed, the controller 27 completes preparation for the exposure operation after the flushing operation for the selected line is completed, instead of immediately stopping application of the drive signal and preparing for the exposure operation.

With this method, it is possible to prevent the automatic exposure request signal auto exp req from being unintentionally generated as the condition of the light detector or the X-ray detector is changed by vibration due to contact between the X-ray imaging apparatus 20 and a patient.

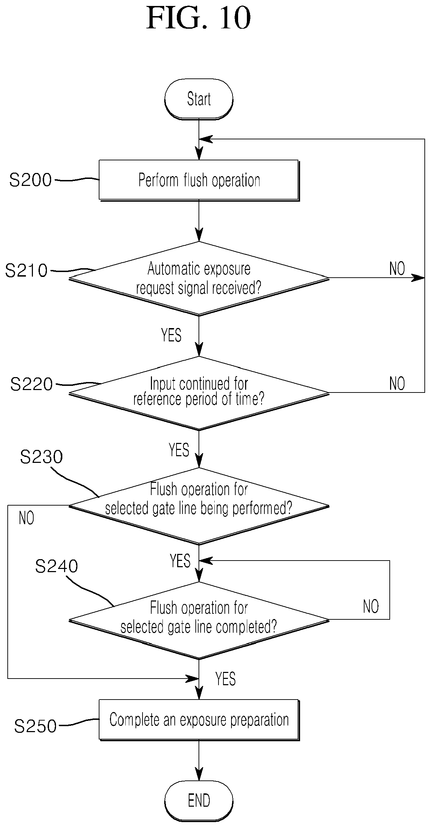

Fig. 10 is a flowchart of an X-ray imaging method according to another exemplary embodiment which will be described with respect to differences thereof from the embodiment shown in Fig. 9.

As shown in Fig. 10, the controller 27 controls the gate driver 24 and the readout unit 25 to sequentially select lines of the image detector 23, and performs a flushing operation for flushing dark currents accumulated in the selected line (S200).

Then, the controller 27 checks whether an automatic exposure request signal auto_exp_req is received from the automatic exposure request signal generator 30 (S210).

When the automatic exposure request signal auto exp req is received from the automatic exposure request signal generator 30, the controller 27 determines whether the automatic exposure request signal auto exp req is continuously input for a predetermined reference period of time (S220).

When the automatic exposure request signal auto exp req is continuously input for the reference period of time, it is determined that the automatic exposure request signal auto exp req is not unintentionally generated by noise, and thus the controller 27 determines that the automatic exposure request signal auto exp req is valid.

On the other hand, when the automatic exposure request signal auto exp req is not continuously input for the reference period of time, it is determined that the automatic exposure request signal auto_exp_req is unintentionally generated by noise, and thus the controller 27 returns to the flushing operation (S200) without performing exposure operation.

If it is determined that the automatic exposure request signal auto exp req is valid, the controller 27 determines whether the flushing operation is performed and completes the preparation procedure for the exposure operation (S230, S240, S250), which is the same as the procedure (S140, S150, S160) in the foregoing embodiment, and repeated descriptions thereof will be omitted.

With this method, it is possible to prevent the automatic exposure request signal auto_exp_req from being unintentionally generated as impulse or noise signals are amplified.

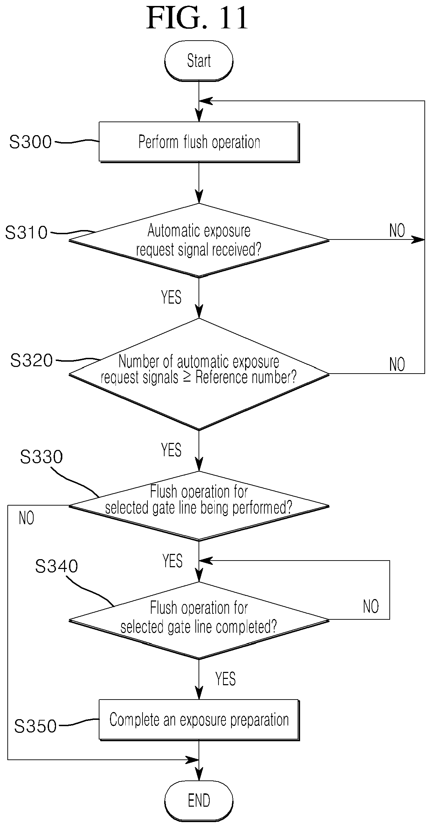

Fig. 11 is a flowchart of an X-ray imaging method according to a further exemplary embodiment which may be applied to a case where the plurality of automatic exposure request signal generators 30 are provided.

As shown in Fig. 11, the controller 27 controls the gate driver 24 and the readout unit 25 to sequentially select lines of the image detector 23, and performs a flushing operation for flushing dark currents accumulated in a selected line (S300).

Then, the controller 27 checks whether an automatic exposure request signal auto exp req is received from the automatic exposure request signal generator 30 (S310), and determines whether a predetermined reference number or more of automatic exposure request signals auto_exp_req are simultaneously received from the plurality of automatic exposure request signal generators 30 (S320).

Here, the expression "simultaneously received" does not mean only the case of receiving the automatic exposure request signals at the exact same time, but may include difference within an allowable range in which a number of automatic exposure request signals caused by the same reason are received at the same time.

If the number of automatic exposure request signals auto exp req simultaneously received by the controller 27 is more than or equal to the predetermined reference number, the controller 27 determines that the automatic exposure request signals auto_exp_req are valid.

On the other hand, if the number of automatic exposure request signals auto_exp_req simultaneously received by the controller 27 is less than the reference number, or if a predetermined reference number or more of automatic exposure request signals auto_exp_req are not simultaneously received, it is determined that the automatic exposure request signals auto_exp_req are invalid, and thus the controller 27 returns to the flushing operation (S300) without performing exposure operation.

If it is determined that the automatic exposure request signals auto exp req are valid, the controller 27 determines whether the flushing operation is performed and completes the preparation procedure for the exposure operation (S330, S340, S350), which is the same as the procedure (S140, S150, S160) in the foregoing embodiment, and repeated descriptions thereof will be omitted.

Meanwhile, in Figs. 9 to 11, the references for determining the validity of the automatic exposure request signal auto_exp_req based on the input vibration from the vibration sensor 29, the continuous time of receiving the automatic exposure request signal auto exp req, and the number of automatic exposure request signals auto exp req received from the plurality of automatic exposure request signal generators 30 may be selectively combined.

For example, the controller 27 may determine that the automatic exposure request signal auto exp req is valid if the input vibration from the vibration sensor 29 is less than or equal to the reference vibration, the automatic exposure request signal auto exp req is continuously input for the reference period of time, and a reference number or more of automatic exposure request signals auto exp req are simultaneously received from the plurality of automatic exposure request signal generators 30.

Fig. 12 is a flowchart of a high-speed a flushing operation by adjusting bias voltage applied to an image detector and controlling a gate driver and a readout unit at high speed, in the X-ray imaging method according to the exemplary embodiment.

As shown in Fig. 12, the controller 27 controls the gate driver 24 and the readout unit 25 to sequentially select lines of the image detector 23, and performs a flushing operation for flushing dark currents accumulated in a selected line (S400).

Then, the controller 27 checks whether an automatic exposure request signal auto exp req is received from the automatic exposure request signal generator 30 (S410).

When the automatic exposure request signal auto exp req is received from the automatic exposure request signal generator 30, the controller 27 determines whether the automatic exposure request signal auto exp req is valid (S420). Here, validity of the automatic exposure request signal auto exp req may be determined based on the methods described with reference to Figs. 9 to 11.

If it is determined that the automatic exposure request signal auto_exp_req is valid, the controller 27 determines whether the drive signal is applied to the pixel of the line selected by the gate driver 24 and the flushing operation is being performed (S430). That is, the controller 27 determines whether the drive signal applied to the selected line is activated.

Then, if it is determined that the drive signal is applied to the pixel of the selected line and the flushing operation is being performed, the controller 27 determines whether the flushing operation for the selected line is terminated (S440).

Then, when the flushing operation regarding the selected line is terminated, the controller 27 prepares for exposure operation (S450) and performs the exposure operation (S460) and a readout operation (S470).

As described above, when the exposure operation is performed, an X-ray emitted from the X-ray generator 10 is converted into visible light, so that electric charges are charged proportional to the intensity of the visible light in a TFT of each of the pixels constituting the image detector 23.

Then, when the readout operation is performed, the levels of the electric charges in the TFTs of the respective pixels are sequentially read out in a unit of line, thereby providing analog data about the X-ray image.

After completing the readout operation, the controller 27 controls the gate driver 24 and the readout unit 25 at high speed, and adjust bias voltage applied to the TFTs of the respective pixels constituting the image detector 23, thereby performing the flushing operation at high speed (S480).

Specifically, the controller 27 applies the bias voltage to the TFTs constituting the image detector 23 and the drive signal to the gate driver 24 and the readout unit 25 according to a predetermined control pattern at a time point when the readout operation is terminated, thereby allowing the flushing operation to be performed at high speed.

Here, the term "control pattern" refers to a mode of adjusting the bias voltage and the drive signal such that the flushing operation can be performed at high speed, in which the bias voltage is converted from a negative voltage to a positive voltage in various gradients and converted again from the positive voltage to the negative voltage.

However, the control pattern may be variously defined for adjusting the speed of the flushing operation according to a designer intention or product specifications.

In this way, when the flushing operation is carried out at high speed, it is possible to rapidly stabilize the image detector 23 and reduce the preparation time for recapturing. Accordingly, a valid automatic exposure request signal is generated even in the case where recapturing is performed within a relatively short time.

Thus, the X-ray imaging apparatus according to the present invention may verify validity of an automatic exposure request signal when capturing an X-ray image using the automatic exposure request signals generated through detection of an X-ray emitted from the X-ray generator, thereby preventing unintentional generation of the automatic exposure request signal due to vibration, temperature change, noise, etc.

Also, according to the present invention, it is possible to prevent unintentional generation of the automatic exposure request signal, thereby improving reliability of an X-ray imaging operation and X-ray images obtained thereby.

## Claims

1. An X-ray imaging apparatus (20) comprising
a scintillator panel (22) which converts an X-ray emitted from an X-ray generator into visible light;
an image detector (23) which includes a plurality of pixels arranged in a matrix, and charges the plurality of pixels with electric charges proportional to intensity of the visible light converted by the scintillator panel;
a gate driver (24) which selects a line of the image detector and applies a drive signal to pixels of the selected line;
an automatic exposure request signal generator (30) which detects the X-ray emitted from the X-ray generator and generates an automatic exposure request signal; and
a controller (27) which verifies validity of the automatic exposure request signal upon receiving the automatic exposure request signal from the automatic exposure request signal generator, and controls a time point for exposure operation according to states of the drive signal when it is determined that the automatic exposure request signal is valid; **characterized in that**
the X-ray imaging apparatus comprises a plurality of automatic exposure request signal generators (30),
wherein the automatic exposure request signal generators of the plurality of automatic exposure request signal generators are disposed at locations corresponding to a middle portion and corners of the image detector, and the controller (27) determines that the automatic exposure request signal is valid when receiving automatic exposure request signals, the number of which is more than or equal to a reference number, from the plurality of the automatic exposure request signal generators (30), and
wherein the automatic exposure request signal generators (30) comprise:
a light to electric signal converter, wherein the light to electric signal converter consists of:
a light converter which converts the X-ray emitted from the X-ray generator into the visible light and a light detector which detects the visible light converted by the light converter and converts the visible light into an electric signal; and
a light detector which detects visible light converted by the scintillator panel and converts the visible light into an electric signal or
an X-ray detector which detects an X-ray emitted from the X-ray generator and converts the X-ray into an electric signal;
and wherein the automatic exposure request signal generators (30) further comprise:
an amplifier which amplifies the electric signal converted by the light detector or by the X-ray detector; and
a trigger conversion circuit which converts the electric signal amplified by the amplifier into a digital signal and generates the automatic exposure request signal; and
an offset compensator which compensates for an offset of the amplifier to maintain the electric signal amplified by the amplifier to be less than or equal to a threshold voltage.

2. The X-ray imaging apparatus according to claim 1, further comprising:
a vibration sensor for detecting external vibration,
wherein the controller determines that the automatic exposure request signal is valid when vibration input from the vibration sensor is less than or equal to reference vibration.

3. The X-ray imaging apparatus according to claim 1, wherein the controller determines that the automatic exposure request signal is valid when the automatic exposure request signal is continuously input for a reference period of time.

## Patentansprüche

1. Röntgenbildaufnahmevorrichtung (20) umfassend
ein Szintillatorpanel (22), das einen von einem Röntgengenerator ausgesendeten Röntgenstrahl in sichtbares Licht umwandelt;
einen Bilddetektor (23), der eine Mehrzahl von in einer Matrix angeordneten Pixeln umfasst und die Mehrzahl von Pixeln mit elektrischen Ladungen auflädt, die zur Intensität des durch das Szintillatorpanel umgewandelten sichtbaren Lichts proportional ist;
einen Gate-Driver (24), der eine Linie des Bilddetektors auswählt und ein Drive-Signal an Pixel der ausgewählten Linie anlegt;
einen Generator von Aufforderungssignalen zur automatischen Belichtung (30), der den von dem Röntgengenerator ausgesendeten Röntgenstrahl detektiert und ein Aufforderungssignal zur automatischen Belichtung erzeugt; und
eine Steuerung (27), die die Gültigkeit des Aufforderungssignals zur automatischen Belichtung verifiziert, nachdem sie das Aufforderungssignal zur automatischen Belichtung von dem Generator von Aufforderungssignalen zur automatischen Belichtung empfangen hat und einen Zeitpunkt für den Belichtungsvorgang gemäß Zuständen des Drive-Signals steuert, wenn bestimmt wird, dass das Aufforderungssignal zur automatischen Belichtung gültig ist; **dadurch gekennzeichnet, dass**
die Röntgenbildaufnahmevorrichtung eine Mehrzahl von Generatoren von Aufforderungssignalen zur automatischen Belichtung (30) umfasst,
wobei die Generatoren von Aufforderungssignalen zur automatischen Belichtung der Mehrzahl von Generatoren von Aufforderungssignalen zur automatischen Belichtung an Stellen angeordnet sind, die einem mittleren Abschnitt und Ecken des Bilddetektors entsprechen, und die Steuerung (27) bestimmt, dass das Aufforderungssignal zur automatischen Belichtung gültig ist, wenn Aufforderungssignale zur automatischen Belichtung empfangen werden, deren Anzahl höher als oder gleich einer Referenzanzahl der Mehrzahl der Generatoren von Aufforderungssignalen zur automatischen Belichtung (30) ist, und
wobei die Generatoren von Aufforderungssignalen zur automatischen Belichtung (30) Folgendes umfassen:
einen Wandler von Licht in ein elektrisches Signal, wobei der Wandler von Licht in ein elektrisches Signal aus Folgendem besteht:
einen Lichtwandler, der den von dem Röntgengenerator ausgesendeten Röntgenstrahl in das sichtbare Licht wandelt, und einen Lichtdetektor, der das durch den Lichtwandler umgewandelte sichtbare Licht detektiert und das sichtbare Licht in ein elektrisches Signal umwandelt; und
einen Lichtdetektor, der von dem Szintillatorpanel umgewandeltes sichtbares Licht detektiert und das sichtbare Licht ein ein elektrisches Signal umwandelt, oder
einen Röntgendetektor, der einen von dem Röntgengenerator ausgesendeten Röntgenstrahl detektiert und den Röntgenstrahl in ein elektrisches Signal umwandelt;
und wobei die Generatoren von Aufforderungssignalen zur automatischen Belichtung (30) Folgendes umfassen:
einen Verstärker, der das durch den Lichtdetektor oder durch den Röntgendetektor umgewandelte elektrische Signal verstärkt; und
eine Triggerwandlungsschaltung, die das durch den Verstärker verstärkte Signal in ein digitales Signal umwandelt und das Aufforderungssignal zur automatischen Belichtung erzeugt; und
einen Versatzkompensator, der einen Versatz des Verstärkers kompensiert, um das durch den Verstärker verstärkte elektrische Signal auf einen Wert, der kleiner als oder gleich einer Schwellenspannung ist, zu kompensieren.

2. Röntgenbildaufnahmevorrichtung nach Anspruch 1, zusätzlich umfassend:
einen Vibrationssensor zum Detektieren einer externen Vibration,
wobei die Steuerung bestimmt, dass das Aufforderungssignal zur automatischen Belichtung gültig ist, wenn die Vibrationseingabe des Vibrationssensors kleiner als oder gleich einer Referenzvibration ist.

3. Röntgenbildaufnahmevorrichtung nach Anspruch 1, wobei die Steuerung bestimmt, dass das Aufforderungssignal zur automatischen Belichtung gültig ist, wenn das Aufforderungssignal zur automatischen Belichtung kontinuierlich eine Eingabe für einen Referenzzeitraum ist.

## Revendications

1. Dispositif d'imagerie à rayons X (20) comprenant
un panneau scintillateur (22) qui convertit un rayon X émis par un générateur de rayons X en lumière visible ;
un détecteur d'image (23) qui comprend une pluralité de pixels disposés dans une matrice, et charge la pluralité de pixels avec des charges électriques proportionnelles à l'intensité de la lumière visible convertie par le panneau de scintillateur ;
un pilote de grille (24) qui sélectionne une ligne du détecteur d'image et applique un signal de commande aux pixels de la ligne sélectionnée ;
un générateur de signal de demande d'exposition automatique (30) qui détecte les rayons X émis par le générateur de rayons X et génère un signal de demande d'exposition automatique ; et
un contrôleur (27) qui vérifie la validité du signal de demande d'exposition automatique lors de la réception du signal de demande d'exposition automatique provenant du générateur de signal de demande d'exposition automatique et contrôle un point de temps pour le fonctionnement d'exposition en fonction des états du signal de commande lorsqu'il est déterminé que le signal de demande d'exposition automatique est valide ; **caractérisé en ce que**
le dispositif d'imagerie à rayons X comprend une pluralité de générateurs de signal de demande d'exposition automatique (30),
dans lequel les générateurs de signal de demande d'exposition automatique de la pluralité de générateurs de signal de demande d'exposition automatique sont disposés à des emplacements correspondant à une portion centrale et des coins du détecteur d'image, et le contrôleur (27) détermine que le signal de demande d'exposition automatique est valide lors de la réception de signaux de demande d'exposition automatique dont le nombre est supérieur ou égal à un nombre de référence, à partir de la pluralité de générateurs de signal de demande d'exposition automatique (30), et
dans lequel les générateurs de signal de demande d'exposition automatique (30) comprennent :
un convertisseur de lumière en signal électrique, ledit convertisseur de lumière en signal électrique étant formé par :
un convertisseur de lumière qui convertit les rayons X émis par le générateur de rayons X en lumière visible et un détecteur de lumière qui détecte la lumière visible convertie par le convertisseur de lumière et convertit la lumière visible en un signal électrique ; et
un détecteur de lumière qui détecte la lumière visible convertie par le panneau scintillateur et convertit la lumière visible en un signal électrique ou
un détecteur de rayons X qui détecte un rayon X émis par le générateur de rayons X et convertit le rayon X en un signal électrique ;
et dans lequel les générateurs de signal de demande d'exposition automatique (30) en outre comprennent :
un amplificateur qui amplifie le signal électrique converti par le détecteur de lumière ou par le détecteur de rayons X ; et
un circuit de conversion à déclenchement qui convertit le signal électrique amplifié par l'amplificateur en un signal numérique et génère le signal de demande d'exposition automatique ; et
un compensateur de décalage qui compense un décalage de l'amplificateur afin de maintenir le signal électrique amplifié par l'amplificateur comme étant inférieur ou égal à une tension de seuil.

2. Dispositif d'imagerie à rayons X selon la revendication 1, comprenant en outre :
un capteur de vibration pour détecter une vibration externe,
le contrôleur déterminant que le signal de demande d'exposition automatique est valide lorsque l'entrée de vibration provenant du capteur de vibration est inférieure ou égale à la vibration de référence.

3. Dispositif d'imagerie à rayons X selon la revendication 1, dans lequel le contrôleur détermine que le signal de demande d'exposition automatique est valide lorsque le signal de demande d'exposition automatique est entré de manière continue pendant une période de temps de référence.
